Europäisches Patentamt

European Patent Office

Office européen des brevets

(11) Publication number: **0 036 145**
**B1**

(12) **EUROPEAN PATENT SPECIFICATION**

(45) Date of publication of patent specification: **29.05.85**

(21) Application number: **81101644.3**

(22) Date of filing: **06.03.81**

(51) Int. Cl.⁴: **A 61 K 31/62,** A 61 K 31/60,
A 61 K 31/66, A 61 K 31/52,
A 61 K 31/71, A 61 K 45/06,
A 61 K 47/00 // (A61K31/71,
31:66, 31:60, 31:52, 31:19)

(54) **An orally administered drug form comprising a polar bioactive agent and an adjuvant.**

(30) Priority: **07.03.80 US 128101**

(43) Date of publication of application:
**23.09.81 Bulletin 81/38**

(45) Publication of the grant of the patent:
**29.05.85 Bulletin 85/22**

(84) Designated Contracting States:
**AT BE CH DE FR GB IT LI LU NL SE**

(56) References cited:
**DE-C- 340 744**
**FR-A-2 042 342**
**FR-A-2 124 101**
**FR-A-2 258 171**
**FR-A-2 293 938**
**FR-A-2 379 285**
**FR-M- 1 650**
**GB-A- 905 092**

(73) Proprietor: **INTERx RESEARCH CORPORATION**
**2201 West 21st Street**
**Lawrence Kansas 66044 (US)**

(72) Inventor: **Higuchi, Takeru**
**2811 Schwarz Road**
**Lawrence Kansas 66044 (US)**
Inventor: **Nishihata, Toshiaki**
**2428 Redbud Lane**
**Lawrence Kansas 66044 (US)**

(74) Representative: **Abitz, Walter, Dr.-Ing. et al**
**Abitz, Morf, Gritschneder P.O. Box 86 01 09**
**D-8000 München 86 (DE)**

(56) References cited:
**ROTE LISTE, 1961, Cantor, Aulendorf/Württ.,
DE**
**ROTE LISTE, 1963, Cantor, Aulendorf/Württ.,
DE**
**Dictionnaire VIDAL, 1961 Office de
Vulgarisation Pharmaceutique, Paris, FR**
**UNLISTED DRUGS, vol. 19, October 1967
Chatham, N.J. US**
**UNLISTED DRUGS, vol. 14, november 1962
Chatham, N.J. US**

UNLISTED DRUGS, vol. 21, july 1969 Chatham, N.J. US

MODERN DRUG ENCYCLOPEDIA, eighth edition, The Rueben H. Donnelley Corp., 1961 New York, US

CHEMICAL ABSTRACTS, vol. 68, no. 23, 03-06-1968, page 9875, column 2, abstract 102325f, Columbus, Ohio, US, KONSTANTY WISNIEWSKI et al.: "Effect of insulin on the transport and the analgesic action of sodium salicylates"

CHEMICAL ABSTRACTS, vol. 84, no. 25, 21-06-1976, page 15, column 1, abstract 173579h, Columbus, Ohio, US, E. BEUBLER et al.: "Methylxanthines and intestinal drug absorption"

UNLISTED DRUGS, vol. 20, september 1968, Chatham, N.J. US

UNLISTED DRUGS, vol. 24, december 1972 Chatham, N.J. US

UNLISTED DRUGS, vol. 28, august 1976 Chatham, N.J. US

**Description**

Background of the invention
Field of the invention

The present invention relates to a drug form with enhanced delivery of polar bioactive agents which normally by this route are slowly absorbed.

As employed in this application, the term "polar bioactive agents" refers to those therapeutic substances which, due to their polar nature, are slowly absorbed from the gastrointestinal tract and include xanthines, polyhydroxylic compounds, zeitterions, polypeptides, organic anions and related chemical compounds.

Description of the prior art

It is well known to the art that a number of bioactive agents are so polar that they are only slowly absorbed from the gastrointestinal tract. Consequently, these agents, on the basis of the current art, must be administered by the intravenous or intramuscular route or in excessively large oral doses in order to attain clinical efficacy. The β-lactam antibiotics and the glycosidic antibiotics are two examples of bioactive agents which are slowly absorbed by the oral route. Similarly, there are a number of other polar bioactive agents such as the xanthines, antitumour agents, narcotic analgesics, agents which contain organic anions, polyhydroxylic agents and polypeptides which, due to their hydrophilic nature, are also slowly absorbed from the gastrointestinal tract. The hydrophilic, polar nature of these agents precludes their rapid absorption so that even the small percentage which is absorbed is subject to a long residency time in the gastrointestinal environment where both acidic and enzymatic degradation contribute to their poor bioavailability. It is therefore clear that any factor which enhances the rate of absorption will demonstrate improved clinical efficacy.

Many attempts have been made to improve the oral absorption of these polar bioactive agents. The degradation caused by the gastric acid and enzymes can be partially overcome by coating. This process in some instances can lead to some enhanced oral absorption, but in no case does it allow complete absorption. Other approaches center on the reduction of the hydrophilicity by preparing a chemical derivative which is more lipophilic. The more lipophilic derivative is more rapidly absorbed so that the residency time in the degrading gastric medium is minimized.

In spite of the numerous attempts to prepare a dosage form of these polar bioactive agents, there still exists a clear and present need for a novel method to enhance the oral absorption of polar bioactive agents. Said method would permit the oral use of a number of agents containing organic anions, polyhydroxy agents and polypeptides, and would provide an improved oral dosage form for xanthines, narcotic analgesics and a number of other agents.

Compositions comprising polar bioactive compounds and variously salicylamide, calcium salicylate, sodium gentisate and sodium salicylate are known from Rote Liste 1961, Contor, Aulendorf/Württ.; Dictionaire Vidal, 1961 Office de Vulgarisation Pharmaceutique, Paris; Unlisted Drugs Vol. 19 November 1967 p. 138e, Unlisted Drugs Vol. 14 November 1962 p. 105g, Unlisted Drugs Vol. 21 July 1969 p. 99c, Modern Drug Encyclopedia 8th Edn. 1961 p. 279. FR—A—2 124 101 describes capsules containing salicylamide together with glyceryl guaiacolate. FR—A—2 042 342 describes oral compositions comprising methyl salicylamide with e.g. phenylbutazone.

FR—A—2 293 938 describes compositions for oral administration comprising alkali salts of salicylic acid with various polar bioactive compounds, e.g., indomethacine, acetylsalicylic acid, phenylbutazone and niflumic acid. FR—A—2 258 171 describes oral compositions comprising steroids and salicylic acid, gentisic acid and their salts with bases. FR—M—1 650 describes oral compositions comprising tetracycline with inter alia diethyl 2,5-dihydroxyphenylphosphonate and shikimic acid. GB—A—905 092 describes oral compositions comprising tetracycline antibiotics, in particular tetracycline itself (claim 3) with 2-naphthol-8-sulphonic acid and 1-naphthol-4-sulphonic acid. However, none of these compositions has been prepared in order to enhance the oral absorption of the contained polar bioactive agent.

Summary of the invention

Accordingly, a major object of this invention is to provide a drug form for oral use with enhanced oral absorption of polar bioactive agents.

The foregoing object is readily attained by a drug form for oral use comprising a polar bioactive agent and an adjuvant comprising a hydroxyaryl acid selected from salicylic acid; 5-methoxysalicylic acid; 3-methylsalicylic acid; 5-tert-octylsalicylic acid; 3-tert-butyl-6-methylsalicylic acid; 3-tert-butyl-5-methylsalicylic acid; 5-bromosalicylic acid; 3,5-diiodosalicylic acid; 5-trifluoromethyl-2-hydroxybenzoic acid; 5-butoxysalicylic acid and 5-chlorosalicylic acid; or salt thereof; in amounts effective in enhancing oral absorption of the polar bioactive agent, with the exception of:

salicylic acid in combination with
a) aminophenazone, caffeine and 2-methyl-6-methylheptene;
b) theophylline, phenyl ethyl barbituric acid and reserpine;
c) acetyl salicylic acid;
d) antipyrine, caffeine, opium and coca;

e) thein and antipyrine;

f) sodium iodide plus colchicine or scilla or aconite;

g) antipyrine, salicylamide, caffeine, vitamin $B_1$ and adenosine;

h) acetaminophen and caffeine;

i) phenylpropanol amine and caffeine;

j) dihydrocodeinone bitartrate, chlorotrimeton maleate, sodium citrate, caffeine and glyceryl guaiacolate;

k) indomethacin;

l) niflumic acid;

m) phenylbutazone;

n) 17-acetoxy-6-α-methyl-4-α-pregnene-3,20-dione and/or 19-ethynyl-α-testosterone-enol-cyclopentyl acetate;

and with the exception of a combination of phenylquinoline carboxylic acid, acetyl salicylic acid, calcium salicylate, caffeine and phenacetine.

The amount of hydroxyaryl acids or salt thereof used in the drug forms may vary over a wide range; in general, the identity and the amount of the hydroxyaryl acid or salt thereof is used in connnection with the drug in order to be effective in enhancing the absorption rate of the drug from the gastrointestinal compartment into the bloodstream. The effectiveness of the hydroxyarylacid adjuvants becomes significant at local concentration exceeding 0.01% at the absorption site. Their use at a dosage whereby their concentration at the absorption site exceeds 5% is not recommended because of the local irritating effect on the tissue.

The polar bioactive agents which can be used according to the invention encompass a variety of therapeutic agents such as the xanthines, triamterene and theophylline, the antitumor agents, 5-fluoroouridinedeoxyriboside, 6-mercaptopurinedeoxyriboside, vidarabine, the narcotic analgesics, hydromorphone, cyclazine, pentazocine, bupomorphine, the compounds containing organic anions, heparin, prostaglandins and prostaglandin-like compounds, cromolyn sodium, carbenoxolone, the polyhydroxylic compounds, dopamine, dobutamine, 1-dopa, α-methyldopa, the polypeptides, angiotensin antagonists, bradykinin, insulin, ACTH, enkaphaline, endorphin, somatostatin, secretin and the miscellaneous compounds such as tetracyclines, bromocriptine, lidocaine, cimetidine or any related compounds. The quantity of these polar bioactive agents necessary for preparing the drug form could vary over a wide range, but would normally be regulated by that quantity necessary to comprise the therapeutically effective dosage form.

The following combinations of polar bioactive agents related compounds and hydroxyaryl or hydroxy aralkyl acids were given to beagle dogs by gavage. The urine was collected by catheterization and blood by venous puncture.

| Example | Polar bioactive agent | Hydroxyaryl or hydroxyaralkyl acid |
|---------|----------------------|------------------------------------|
| 1 | triamterene | sodium salicylate |
| 2 | theophylline | salicylic acid |
| 3 | cyclazine | sodium salicylate |
| 4 | pentazocine | salicylic acid |
| 5 | dopamine | salicylic acid |
| 6 | saralasin acetate | sodium salicylate |
| 7 | enkephalin | salicylic acid |
| 8 | endorphin | sodium salicylate |
| 9 | chlorotetracycline | salicylic acid |

The drug forms of this invention are suitably administered in oral dosage form, such as by tablet or capsule, by combining the polar bioactive agent in a therapeutic amount and the hydroxyaryl acid or salt thereof in a sufficient quantity to be effective to enhance oral delivery with an oral pharmaceutically acceptable inert carrier, such as lactose, starch (pharmaceutical grade), dicalcium phosphate, calcium sulfate, Kaolin, mannitol and powdered sugar. In order to reduce the irritation in the stomach, the preferred dose form of the hydroxyaryl or hydroxyaralkyl acid should be a pharmaceutically acceptable salt and the drug form should be designed to release the polar bioactive agent and the hydroxyaryl or hydroxyaralkyl acid salt beyond the pylorus. In addition, when required, suitable binders, lubricants, disintegrating agents,

4

and coloring agents can also be added. Typical binders include, without limitation, starch, gelatin, sugars such as sucrose, molasses, and lactose, natural and synthetic gums, such as acacia, sodium alginate, extract of Irish moss, carboxymethylcellulose, methylcellulose, and polyvinylpyrrolidone, polyethylene glycol, ethylcellulose and waxes. Typical lubricants for use in these dosage forms can include, without limitation, boric acid, sodium benzoate, sodium acetate, sodium chloride, leucine and polyethylene glycol. Suitable disintegrators can include, without limitation, starch, methylcellulose, agar, bentonite, cellulose and wood products, alginic acid, guar gum, citris pulp, carboxymethylcellulose, and sodium lauryl sulfate. Optionally, if desired, a conventional, pharmaceutically acceptable dye can be incorporated into oral dosage unit form, e.g., any of the standard FD & C dyes.

Example I
    Typical preparation of enteric-coated tablets continuing adjuvant.

125 mg Methyldopa tablets

| Ingredient | Amount per tablet |
| --- | --- |
| Methyldopa | 125 mg |
| Sodium 5-methoxysalicylate | 250 mg |
| Microcrystalline cellulose | 150 mg |
| Lactose | 95 mg |
| Magnesium stearate | 40 mg |
| Total | 660 mg |

All ingredients except 1/4 of the magnesium stearate were mixed and the material slugged using 1.27 cm flat head punches. The slugs were broken up and passed through a screen with 0.42 mm openings. The remaining magnesium stearate was added and mixed in. Tablets were made with 1.11 cm deep concave punches to a hardness of 10 Kg.
    Coating: the tablets were coated with 11 mg of pre-coat and 32 mg of enteric coating according to the coating procedure described below.

Enteric coating procedure
    Tablets or capsules were placed in a coating pan containing baffles to provide adequate tumbling. A small amount of the coating solution was applied using an air sprayer and the solvents evaporated with a warm air supply directed into the coating pan. This procedure was repeated until the desired amount of coating material was applied. The amount of coating material was determined from the weight gain of a representative group of tablets.
    Coating solutions:
        *Pre-coat:* A film of hydroxypropylmethylcellulose was applied to the tablets followed by an enteric coating.
        *Enteric coat:* A film of hydroxypropylmethylcellulosephthalate was applied.
        *Solutions:* A 5% by weight solution of hydroxypropylmethylcellulose and a 10% by weight solution of hydroxypropylmethylcellulosephthalate in ethanol:methylene chloride (1:1 by weight) were used as the coating solutions.

Example II
    In the like manner of Example I the following amount of various drugs may be incorporated into tablets using the same excipient and adjuvant and preparation technique described above:

| Drug | Amount |
| --- | --- |
| hydrochlorothiazide | 75 mg. |
| Sinemet (carbidopa and levodopa) | 50/200 mg. |
| cyclobenzaprine | 10 mg. |
| diflunisal | 250 mg. |
| indomethacin | 75 mg. |
| methyldopa | 500 mg. |
| sulindac | 200 mg. |
| ibuprofen | 600 mg. |
| naproxen | 250 mg. |
| phenylbutazone | 100 mg. |
| dexamethasone | 4 mg. |
| prednisolone | 25 mg. |
| clonidine | 0.1 mg. |
| propranolol | 40 mg. |
| diazepam | 5 mg. |
| chlorodiazepoxide | 5 mg. |
| furosemide | 60 mg. |
| cephamandole | 1000 mg. |
| nalidixic acid | 1000 mg. |
| haloperidol | 3 mg. |
| captopril | |
| timolol | |
| (−)-1-(cyclopropylmethyl)-4-[3-(trifluoromethylthio)-5H-dibenzo(a,d) cyclohepten-5-ylidene]piperidine hydrochloride | |
| N-[(S)-1-(ethoxycarbonyl)-3-phenylpropyl]-L-alanyl-L-proline maleate | |
| (+)10,11-dihydro-5-methyl-5H-dibenzo[a,d]cyclohepten-5,10-imine oxalate | |
| 1-ethyl-6-fluoro-1,4-dihydro-4-oxo-7-(1-piperazinyl)-3-quinoline-carboxylic acid | |

# 0 036 145

3-fluoro-*D*-alanine and *D*-4-(1-methyl-
3-oxo-1-butenylamino)-
3-isoxazolidinone sodium salt
hemihydrate

*L*-N-(2-oxopiperidin-6-ylcarbonyl)-histidyl-*L*-thazol-
idine-4-carboxamide

N-formimidoyl thienamycin
monohydrate

(6,7-dichloro-2-methyl-2-
phenyl-1-oxo-5-indanyloxy)
acetic acid

The adjuvants may be chosen from the following salts or their acids:
Sodium 5-methoxysalicylate
Sodium salicylate
Sodium 3-methylsalicylate
Sodium 5-methylsalicylate
Sodium 5-tert-octylsalicylate
Sodium 3-tert-butyl-5-methylsalicylate
Sodium 5-bromosalicylate
Sodium 3,5-diiodosalicylate
Sodium 5-trifluoromethyl-2-hydroxybenzoate
Sodium 5-butoxysalicylate
Sodium 5-chlorosalicylate

Example III
Following are also specific examples of polar bioactive agents which can be combined in equivalent ratios or previously described with any one of the hydroxyaryl acids or salts thereof previously mentioned or with those specifically mentioned below.

|  | | Drug | Adjuvant |
|---|---|---|---|
| 1 | | 3,5-diamino-N-(aminoimino-methyl)-6-chloropyrazine-carboxamide (amiloride); | sodium salicylate |
| 2 | | S-α-hydrazino-3,4-dihydroxy-α-methylbenzenepropanoic acid monohydrate (carbidopa); | sodium 5-methoxy salicylate |
| 3 | | 3-hydroxy-α-methyl-L-tyrosine (methyldopa) | sodium salicylate |
| 4 | | (Z)-5-fluoro-2-methyl-1-[[4-(methylsulfinyl)phenyl]methylene]-1H-indene-3-acetic acid (sulindac); | sodium 5-methoxy salicylate |
| 5 | | S-(−)-1-(tert-butylamino)-3-[(4-morpholino-1,2,5-thiadiazol-3-yl)oxy]-2-propanol (timolol); | sodium salicylate |
| 6 | | (−)-1-(cyclopropylmethyl)-4-[3-(trifluoromethylthio)-5H-dibenzo(a,d) cyclohepten-5-ylidene]piperidine hydro-chloride | sodium salicylate |

7

| | Drug | Adjuvant |
|---|---|---|
| 7 | 1-ethyl-6-fluoro-1,4-dihydro-4-oxo-7-(1-piperazinyl)-3-quinoline-carboxylic acid | sodium 5-methoxy salicylate |
| 8 | *L*-N-(2-oxopiperidin-6-yl-carbonyl)-histidyl-*L*-thiazol-indine-4-carboxamide | sodium salicylate |
| 9 | (+)-6-methoxy-α-methyl-2-naphthaleneacetic acid (naproxen) | sodium salicylate |
| 10 | 5-(4-chlorobenzoyl)-1,4-di-methyl-1*H*-pyrrole-2-acetic acid | sodium 5-methoxy salicylate |
| 11 | 4-butyl-1,2-diphenyl-3,5-pyrazolidinedione (phenyl-butazone) | sodium salicylate |
| 12 | 9-fluoro-11β,17,21-tri-hydroxy-16α-methyl pregna-1,4-diene-3,20-dione (dexamethasone) | sodium salicylate |
| 13 | 1-(isopropylamino)-3-(1-naphthyloxy-2-propanol (propanolol) | sodium 5-methoxy salicylate |
| 14 | 7-chloro-N-methyl-5-phenyl-3*H*-1,4-benzodiazepin-2-amine 4-oxide (chlorodiazepoxide) | sodium salicylate |
| 15 | 4-[4-(4-chlorophenyl)-4-hydroxy-1-piperidinyl]-1-(4-fluorophenyl)-1-butazone (haloperidol) | sodium 5-methoxy-salicylate |
| 16 | 1-(3-mercapto-2-methyl-1-oxopropyl)-*L*-proline (captopril) | sodium salicylate |

As already described, the drug form of the present invention with enhanced rate of absorption of polar bioactive agents orally is useful for a wide range of these particular polar bioactive agents. Without limiting the broad applicability, there is also pointed out below a number of these agents for which the invention is particularly useful.

3,5 - diamino - N - (aminoiminomethyl) - 6 - chloropyrazinecarboxamide (amiloride);

6 - chloro - 3,4 - dihydro - 2H - 1,24 - benzothiadiazine - 7 - sulfonamide - 1,1 - dioxide (hydrochlorothiazide);

amiloride hydrochloride and hydrochlorothiazide (Moduretic);

S - α - hydrozino - 3,4 - dihydroxy - α - methylbenzenepropanoic acid monohydrate (carbodopa);

carbidopa and 3 - hydroxy - L - tyrosine (levodopa) (Sinemet);

3 - (5H - dibenzo[a,d]cyclohepten - 5 - ylidene) - N,N - dimethyl - 1 - propanamine (cyclobenzaprine);

2',4' - difluoro - 4 - hydroxy[1,1' - biphenyl] - 3 - carboxylic acid (diflunisal);

1 - (p - chlorobenzoyl) - 5 - methoxy - 2 - methylindole - 3 - acetic acid (indomethacin);

3 - hydroxy - α - methyl - L - tyrosine (methyldopa);

(Z) - 5 - fluoro - 2 - methyl - 1 - [[4 - (methylsulfinyl)phenyl]methylene] - 1H - indene - 3 - acetic aid (sulindac);

S - (−) - 1 - (tert - butylamino) - 3 - [(4 - morpholino - 1,2,5 - thiadiazol - 3 - yl)oxy] - 2 - propanol (timolol);

(−) - 1 - (cyclopropylmethyl) - 4 - [3 - (trifluoromethylthio) - 5*H* - dibenzo(a,d) cyclohepten - 5 - ylidene]piperidine hydrochloride

N - [(S) - 1(ethoxycarbonyl) - 3 - phenylpropyl] - L - alanyl - L - proline maleate

(+)10,11 - dihydro - 5 - methyl - 5*H* - dibenzo[a,d] cyclohepten - 5,10 - imine oxalate

1 - ethyl - 6 - fluoro - 1,4 - dihydro - 4 - oxo - 7 - (1 - piperazinyl) - 3 - quinolinecarboxylic acid

3 - fluoro - *D* - alanine and *D* - 4 - (1 - methyl - 3 - oxo - 1 - butenylamino) - 3 - isoxazolidinone sodium salt hemihydrate

*L* - N - (2 - oxopiperidine - 6 - ylcarbonyl) - histidyl - *L* - thiazolidine - 4 - carboxamide

(6,7 - dichloro - 2 - methyl - 2 - phenyl - 1 - oxo - 5 - indanyloxy) acetic acid

α - methyl - 4 - (2 - methylpropyl)benzeneacetic acid (ibuprofen)

(+) - 6 - methoxy - α - methyl - 2 - naphthaleneacetic acid (naproxen)

5 - (4 - chlorobenzoyl) - 1,4 - dimethyl - 1*H* - pyrrole - 2 - acetic acid

4 - butyl - 1,2 - diphenyl - 3,5 - pyrazolidinedione (phenylbutazone)

9 - fluoro - 11β,17,21 - trihydroxy - 16α - methylpregna - 1,4 - diene - 3,20 - dione (dexamethasone)

11β,17,21,trihydroxypregna - 1,4 - diene - 3,20 - dione (prednisolone)

2 - (2,6 - dichloroanilino) - 2 - imidazoline (clonidine)

1 - (isopropylamino) - 3 - (1 - naphthyloxy) - 2 - propanol (propanolol)

7 - chloro - 1,3 - dihydro - 1 - methyl - 5 - phenyl - 2*H* - 1,4 - benzodiazepin - 2 - one (diazepam)

7 - chloro - N - methyl - 5 - phenyl - 3*H* - 1,4 - benzodiazepin - 2 - amine 4 - oxide (chlordiazepoxide)

5 - (aminosulfonyl) - 4 - chloro - 2 - [(2 - furanylmethyl)amino]benzoic acid (furosemide)

1 - ethyl - 1,4 - dihydro - 7 - methyl - 4 - oxo - 1,8 - naphthyridine - 3 - carboxylic acid (nalidixic acid)

4 - [4 - (4 - chlorophenyl) - 4 - hydroxy - 1 - piperidinyl] - 1 - (4 - fluorophenyl) - 1 - butanone (haloperidol)

1 - (3 - mercapto - 2 - methyl - 1 - oxopropyl) - *L* - proline (captopril)

Any skilled artisan concerned with the subject matter of this invention can prepare these oral dosage forms by simply referring to the oral dosage form preparatory procedure outlined in Remington's Pharmaceutical Sciences, Fifteenth Edition (1975), pages 1576 to 1617 inclusive.

**Claims for the Contracting States: BE, CH, DE, FR, GB, IT, LI, LU, NL, SE:**

1. A drug form for oral use comprising a polar bioactive agent and an adjuvant comprising a hydroxyaryl acid selected from salicylic acid; 5-methoxysalicylic acid; 3-methylsalicylic acid; 5-tert-octylsalicylic acid; 3 - tert - butyl - 6 - methylsalicylic acid; 3 - tert - butyl - 5 - methylsalicylic acid; 5 - bromosalicylic acid; 3,5 - diiodosalicylic acid; 5 - trifluoromethyl - 2 - hydroxybenzoic acid; 5 - butoxysalicylic acid and 5 - chlorosalicylic acid; or salt thereof; in amounts effective in enhancing oral absorption of the polar bioactive agent, with the exception of: salicylic acid in combination with

a) aminophenazone, caffeine and 2 - methyl - 6 - methylheptene;

b) theophylline, phenyl ethyl barbituric acid and reserpine;

c) acetyl salicylic acid;

d) antipyrine, caffeine, opium and coca;

e) theine and antipyrine;

f) sodium iodide plus colchicine or scilla or aconite;

g) antipyrine, salicylamide, caffeine, vitamin $B_1$ and adenosine;

h) acetaminophen and caffeine;

i) phenylpropanol amine and caffeine;

j) dihydrocodeinone bitartrate, chlorotrimeton maleate, sodium citrate, caffeine and glyceryl guaiacolate;

k) indomethacin;

l) niflumic acid;

m) phenylbutazone;

n) 17 - acetoxy - 6 - α - methyl - 4 - α - pregnene - 3,20 - dione and/or 19 - ethynyl - α - testosterone - enol - cyclopentyl acetate;

and with the exception of a combination of phenylquinoline carboxylic acid, acetyl salicylic acid, calcium salicylate, caffeine and phenacetine.

2. The drug form of Claim 1 wherein said polar bioactive agent is xanthines, antitumor agents, narcotic analgesics, biologically active organic anion, polyhydroxylic bioactive compounds, bioactive polypeptides.

3. The drug form of Claim 2 wherein said xanthines is triamterene or theophyiine.

4. The drug form of Claim 2 wherein said antitumor agent is 5 - fluorouridine deoxyriboside, 6 - mercaptopurine deoxyriboside or vidarabine.

5. The drug form of Claim 2 wherein said narcotic analgesic is hydromorphone, cyclazine, pentazocine or bupomorphine.

6. The drug form of Claim 2 wherein said biologically active organic anion is heparin, 15-methyl prostaglandin $E_2$, cromolyn sodium or carbenoxolone.

7. The drug form of Claim 2 wherein said polyhydroxylic bioactive compound is dopamine, dobutamine, 1-dopa, or α-methyldopa.

8. The drug form of Claim 2 wherein said bioactive polypeptide is saralasin acetate, bradykinin, insulin, ACTH, enkephalin, endorphin, somatostatin or secretin.

9. The drug form of Claim 2 wherein said polar bioactive agent is chlorotetracycline, bromocriptine, lidocaine or cimetidine.

10. The drug form of Claim 2 wherein said polar bioactive agent is amiloride, hydrochlorothiazide, moduretic, carbidopa, levodopa, cyclobenzaprine, diflunisal, indomethacin, methyldopa, sulindac, timolol, (−) - 1 - (cyclopropylmethyl)4 - [3 - (trifluoromethylthio) 5H - dibenzo(a,d)cyclohepten - 5 - ylidene]piperidine hydrochloride, N - [(S) - 1 - (ethoxycarbonyl) - 3 - phenylpropyl] - L - alanyl - L - prolinemaleate, (+)10,11 - dihydro - 5 - methyl - 5H - dibenzo[a,d]cyclohepten - 5,10 - imine oxalate, 1 - ethyl - 6 - fluoro - 1,4 - dihydro - 4 - oxo - 7 - (1 - piperazinyl) - 3 - quinoline - carboxylic acid, 3 - fluoro - *D* - alanine and *D* - 4 - (1 - methyl - 3 - oxo - 1 - butenylamino) - 3 - isoxazolidinone sodium salt hemihydrate, *L* - N - (2 - oxopiperidin - 6 - ylcarbonyl)histidyl - *L* - thiazolidine - 4 - carboxamide, (6,7 - dichloro - 2 - methyl - 2 - phenyl - 1 - oxo - 5 - indanyloxy) acetic acid, ibuprofen, naproxen, 5 - (4 - chlorobenzoyl) - 1,4 - dimethyl - 1*H* - pyrrole - 2 - acetic acid, phenylbutazone, dexamethasone, prednisolone, clonidine, propranolol, diazepam, chlorodiazeproxide, furosemide, nalidixic acid, haloperidol, or captopril.

**Claims for the Contracting State: AT**

1. A method for preparing a drug form for oral use comprising combining a polar bioactive agent together with an adjuvant comprising a hydroxyaryl acid selected from salicylic acid; 5 - methoxysalicylic acid; 3 - methylsalicylic acid; 5 - tert - octylsalicylic acid; 3 - tert - butyl - 6 - methylsalicylic acid; 3 - tert - butyl - 5 - methylsalicylic acid; 5 - bromosalicylic acid; 3,5 - diiodosalicylic acid; 5 - trifluoromethyl - 2 - hydroxybenzoic acid; 5 - butoxysalicylic acid and 5 - chlorosalicylic acid; or salt thereof; in an amount effective in enhancing oral absorption of the polar bioactive agent, with the exception of:
salicylic acid in combination with
   a) aminophenazone, caffeine and 2 - methyl - 6 - methylheptene;
   b) theophylline, phenyl ethyl barbituric acid and reserpine;
   c) acetyl salicylic acid;
   d) antipyrine, caffeine, opium and coca;
   e) theine and antipyrine;
   f) sodium iodide plus colchicine or scilla or aconite;
   g) antipyrine, salicylamide, caffeine, vitamin $B_1$ and adenosine;
   h) acetaminophen and caffeine;
   i) phenylpropanol amine and caffeine;
   j) dihydrocodeinone bitartrate, chlorotrimeton maleate, sodium citrate, caffeine and glyceryl guaiacolate;
   k) indomethacin;
   l) niflumic acid;
   m) phenylbutazone;
   n) 17 - acetoxy - 6 - α - methyl - 4 - α - pregnene - 3,20 - dione and/or 19 - ethynyl - α - testosterone - enol - cyclopentyl acetate;
and with the exception of a combination of phenylquinoline carboxylic acid, acetyl salicylic acid, calcium salicylate, caffeine and phenacetine.

2. The method of Claim 1 wherein said polar bioactive agent is xanthines, antitumor agents, narcotic analgesics, biologically active organic anion, polyhydroxylic bioactive compounds or bioactive polypeptides.

3. The method of Claim 2 wherein said xanthines is triamterene or theophylline.

4. The method of Claim 2 wherein said antitumor agent is 5 - fluorouridine deoxyriboside, 6 - mercaptopurine deoxyriboside or vidarabine.

5. The method of Claim 2 wherein said narcotic analgesic is hydromorphone, cyclazine, pentazocine or bupomorphine.

6. The method of Claim 2 wherein said biologically active organic anion is heparin, 15-methyl prostaglandin $E_2$, cromolyn sodium or carbenoxolone.

7. The method of Claim 2 wherein said polyhydroxylic bioactive compound is dopamine, dobutamine, 1-dopa, or α-methyldopa.

8. The method of Claim 2 wherein said bioactive polypeptide is saralasin acetate, bradykinin, insulin, ACTH, enkephalin, endorphin, somatostatin or scretin.

9. The method of Claim 2 wherein said polar bioactive agent is chlorotetracycline, bromocriptine, lidocaine or cimetidine.

10. The method of Claim 2 wherein said polar bioactive agent is: amiloride, hydrochlorothiazide, moduretic, carbidopa, levodopa, cyclobenzaprine, diflunisal, indomethacin, methyldopa, sulindac, timolol, (−) - 1 - (cyclopropylmethyl) 4 - [3 - (trifluoromethylthio) - 5H - dibenzo(a,d)cyclohepten - 5 - ylidene]piperidine hydrochloride, N - [(S) - 1 - (ethoxycarbonyl) - 3 - phenylpropyl] - L - alanyl - L - proline maleate, (+)10,11 - dihydro - 5 - methyl - 5H - dibenzo[a,d] cyclohepten - 5,10 - imine oxalate, 1 - ethyl - 6 - fluoro - 1,4 - dihydro - 4 - oxo - 7 - (1 - piperazinyl) - 3 - quinoline - carboxylic acid, 3 - fluoro - *D* - alanine and *D* - 4 - (1 - methyl - 3 - oxo - 1 - butenylamino) - 3 - isoxazolidinone sodium salt hemihydrate, *L* - N - (2 - oxopiperidin - 6 - ylcarbonyl) - histidyl - *L* - thiazolidine - 4 - carboxamide, (6,7 - dichloro - 2 - methyl - 2 - phenyl - 1 - oxo - 5 - indanyloxy) acetic acid, ibuprofen, naproxen, 5 - (4 - chlorobenzoyl) - 1,4 - dimethyl - 1*H* - pyrrole - 2 - acetic acid, phenylbutazone, dexamethasone, prednisolone, clonidine, propranolol, diazepam, chlorodiazeproxide, furosemide, nalidixic acid, haloperidol, and captopril.

## Patentansprüche für die Vertragsstaat: BE, CH, DE, FR, GB, IT, LI, LU, NL, SE

1. Eine Arzneimittelformulierung zur oralen Verarbreichung mit einem Gehalt an einem polaren, bioaktiven Wirkstoff und einem Hilfsstoff, umfassend eine Hydroxyarylsäure, ausgewählt aus Salicylsäure; 5-Methoxysalicylsäure, 3-Methylsalicylsäure; 5-tert.Octylsalicylsäure; 3-tert.Butyl - 6 - methylsalicylsäure; 3 - tert.Butyl - 5 - methylsalicylsäure; 5 - Bromsalicylsäure; 3,5 - Diiodsalicylsäure; 5 - Trifluormethyl - 2 - hydroxybenzoesäure; 5 - Butoxysalicylsäure und 5-Chlorsalicylsäure; oder einem Salz davon; in zur Erhöhung der oralen Absorption des polaren, bioaktiven Wirkstoffes wirksamen Mengen, mit Ausnahme von:
Salicylsäure in Kombination mit
    a) Aminophenazon, Coffein und 2 - Methyl - 6 - methylhepten;
    b) theophyllin, Phenylethylbarbitursäure und Reserpin;
    c) Acetylsalicylsäure;
    d) Antipyrin, Coffein, Opium und Coca;
    e) Thein und Antipyrin;
    f) Natriumiodid plus Colchicin oder Scilla oder Aconit;
    g) Antipyrin, Salicylamid, Coffein, Vitamin $B_1$ und Adenosin;
    h) Acetaminophen und Coffein;
    i) Phenylpropanolamin und Coffein;
    j) Dihydrocodeinonbitartrat, Chlorotrimetonmaleat, Natriumcitrat, Coffein und Glycerylguajacolat;
    k) Indomethacin;
    l) Nifluminsäure;
    m) Phenylbutazon;
    n) 17 - Acetoxy - 6 - α - methyl - 4 - α - pregnen - 3,20 - dion und/oder 19 - Ethynyl - α - testosteron - enol - cyclopentylacetat;
und mit Ausnahme einer Kombination von Phenylchinolincarbonsäure, Acetylsalicylsäure, Calciumsalicylat, Coffein und Phenacetin.

2. Die Arzneimittelformulierung des Anspruchs 1, worin der genannte polare, bioaktive Wirkstoff Xanthine, Antitumormittel, narkotische Analgetika, biologisch aktives organisches Anion, polyhydroxylische, bioaktive Verbindungen oder bioaktive Polypeptide darstellt.

3. Die Arzneimittelformulierung des Anspruchs 2, worin das genannte Xanthin Triamteren oder Theophyllin ist.

4. Die Arzneimittelformulierung des Anspruchs 2, worin das genannte Antitumormittel 5 - Fluoruridindeoxyribosid, 6 - Mercaptopurindeoxyribosid oder Vidarabin ist.

5. Die Arzneimittelformulierung des Anspruchs 2, worin das genannte narkotische Analgetikum Hydromorphon, Cyclazin, Pentazocin oder Bupomorphine ist.

6. Die Arzneimittelformulierung des Anspruchs 2, worin das genannte biologisch aktive organische Anion Heparin, 15 - Methylprostaglandin $E_2$, Cromolyn-Natrium oder Carbenoxolon ist.

7. Die Arzneimittelformulierung des Anspruchs 2, worin die genannte polyhydroxylische, bioaktive Verbindung Dopamin, Dobutamin, 1 - Dopa oder α - Methyldopa ist.

8. Die Arzneimittelformulierung des Anspruchs 2, worin das genannte bioaktive Polypeptid Saralasinacetat, Bradykinin, Insulin, ACTH, Enkephalin, Endorphin, Somatostatin oder Secretin ist.

9. Die Arzneimittelformulierung des Anspruchs 2, worin der genannte polare, bioaktive Wirkstoff Chlorotetracyclin, Bromocriptin, Lidocain oder Cimetidin ist.

10. Die Arzneimittelformulierung des Anspruchs 2, worin der genannte polare, bioaktive Wirkstoff Amilorid, Hydrochlorothiazid, Moduretic, Carbidopa, Levodopa, Cyclobenzaprin, Diflunisal, Indomethacin, Methyldopa, Sulindac, Timolol, (−) - 1 - (Cylopropylmethyl) - 4 - [3 - (trifluoromethylthio) - 5H - dibenzo(a,d)cyclohepten - 5 - yliden] - piperidinhydrochlorid, N - [(S) - 1 - (Ethoxycarbonyl) - 3 - phenylpropyl] - L - alanyl - L - prolinmaleat, (+)10,11 - Dihydro - 5 - methyl - 5H - dibenzo[a,d]cyclohepten - 5,10 - iminoxalat, 1 - Ethyl - 6 - fluor - 1,4 - dihydro - 4 - oxo - 7 - (1 - piperazinyl) - 3 - chinolin - carbonsäure, 3 - Fluor - D - alanin und D - 4 - (1 - Methyl - 3 - oxo - 1 - butenylamino) - 3 - isoxazolidinon - Natriumsalzhemihydrat, L - N - (2 - Oxopiperidin - 6 - ylcarbonyl) - histidyl - L - thiazolidin - 4 - carboxamid, (6,7 - Dichlor - 2 - methyl - 2 - phenyl - 1 - oxo - 5 - indanyloxy)essigsäure, Ibuprofen, Naproxen, 5 - (4 - Chlorbenzoyl) - 1,4 - dimethyl - 1H - pyrrol - 2 - essigsäure, Phenylbutazon, Dexamethason, Prednisolon, Clonidin, Propranolol, Diazepam, Chlorodiazeproxid, Furosemid, Nalidixinsäure, Haloperidol oder Captopril ist.

### Patentansprüche für den Vertragsstaat: AT

1. Ein Verfahren zur Herstellung einer Arzneimittelformulierung zur oralen Verabreichung, umfassend die Vereinigung eines polaren, bioaktiven Wirkstoffes zusammen mit einem eine Hydroxyarylsäure enthaltenden Hilfsstoff, der aus Salicylsäure; 5-Methoxysalicylsäure; 3-Methylsalicylsäure; 5 - tert.Octylsalicylsäure; 3 - tert.Butyl - 6 - methylsalicylsäure; 3 - tert.Butyl - 5 - methylsalicylsäure; 5 - Bromsalicylsäure; 3,5 - Diiodsalicylsäure; 5 - Trifluormethyl - 2 - hydroxybenzoesäure; 5-Butoxysalicylsäure und 5-Chlorsalicylsäure; oder einem Salz davon ausgewählt ist; in einer zur Erhöhung der oralen Absorption des polaren, bioaktiven Wirkstoffes wirksamen Menge, mit Ausnahme von: Salicylsäure in Kombination mit

a) Aminophenazon, Coffein und 2 - Methyl - 6 - methylhepten;
b) Theophyllin, Phenylethylbarbitursäure und Reserpin;
c) Acetylsalicylsäure;
d) Antipyrin, Coffein, Opium und Coca;
e) Thein und Antipyrin;
f) Natriumiodid plus Colchicin oder Scilla oder Aconit;
g) Antipyrin, Salicylamid, Coffein, Vitamin $B_1$ und Adenosin;
h) Acetaminophen und Coffein;
i) Phenylpropanolamin und Coffein;
j) Dihydrocodeinonbitartrat, Chlorotrimetonmaleat, Natriumcitrat, Coffein und Glycerylguajacolat;
k) Indomethacin;
l) Nifluminsäure;
m) Phenylbutazon;
n) 17 - Acetoxy - 6 - α - methyl - 4 - α - pregnen - 3,20 - dion und/oder 19 - Ethynyl - α - testosteron - enol - cyclopentylacetat;

und mit Ausnahme einer Kombination von Phenylchinolincarbonsäure, Acetylsalicylsäure, Calciumsalicylat, Coffein und Phenacetin.

2. Das Verfahren des Anspruchs 1, worin der genannte polare, bioaktive Wirkstoff Xanthine, Antitumormittel, narkotische Analgetika, biologisch aktives organisches Anion, polyhydroxylische, bioaktive Verbindungen oder bioaktive Polypeptide darstellt.

3. Das Verfahren des Anspruchs 2, worin das genannte Xanthin Triamteren oder Theophyllin ist.

4. Das Verfahren des Anspruchs 2, worin das genannte Antitumormittel 5-Fluoruridindeoxyribosid, 6-Mercaptopurindeoxyribosi oder Vidarabin ist.

5. Das Verfahren des Anspruchs 2, worin das genannte narkotische Analgetikum Hydromorphon, Cyclazin, Pentazocin oder Bupomorphin ist.

6. Das Verfahren des Anspruchs 2, worin das genannte biologisch aktive organische Anion Heparin, 15-Methylprostaglandin $E_2$, Cromolyn-Natrium oder Carbenoxolon ist.

7. Das Verfahren des Anspruchs 2, worin die genannte polyhydroxylische, bioaktive Verbindung Dopamin, Dobutamin, 1-Dopa oder α-Methyldopa ist.

8. Das Verfahren des Anspruchs 2, worin das genannte bioaktive Polypetid Saralasinacetat, Bradykinin, Insulin, ACTH, Enkephalin, Endorphin, Somatostatin oder Secretin ist.

9. Das Verfahren des Anspruchs 2, worin der genannte polare, bioaktive Wirkstoff Chlorotetracyclin, Bromocriptin, Lidocain oder Cimetidin ist.

10. Das Verfahren des Anspruchs 2, worin der genannte polare, bioaktive Wirkstoff Amilorid, Hydrochlorothiazid, Moduretic, Carbidopa, Levodopa, Cyclobenzaprin, Diflunisal, Indomethacin, Methyldopa, Sulindac, Timolol, (−) - 1 - (Cyclopropylmethyl) - 4 - [3 - (trifluormethylthio) - 5H - dibenzo(a,d)cyclohepten - 5 - yliden] - piperidinhydrochlorid, N - [(S) - 1 - (Ethoxycarbonyl) - 3 - phenylpropyl] - L - alanyl - L - prolinmaleat, (+)10,11 - Dihydro - 5 - methyl - 5H - dibenzo[a,d]cyclohepten - 5,10 - iminoxalat, 1 - Ethyl - 6 - fluor - 1,4 - dihydro - 4 - oxo - 7 - (1 - piperazinyl) - 3 - chinolin - carbonsäure, 3 - Fluor - D - alanin und D - 4 - (1 - Methyl - 3 - oxo - 1 - butenylamino) - 3 - isoxazolidinon-Natriumsalzhemihydrat, L - N - (2 - Oxopiperidin - 6 - ylcarbonyl) - histidyl - L - thiazolidin - 4 - carboxamid, (6,7 - Dichlor - 2 - methyl - 2 - phenyl - 1 - oxo - 5 - indanyloxy)essigsäure, Ibuprofen, Naproxen, 5 - (4 - Chlorbenzoyl) - 1,4 - dimethyl - 1H - pyrrol - 2 - essigsäure, Phenylbutazon, Dexamethason, Prednisolon, Clonidin, Propranolol, Diazepam, Chlorodiazeproxid, Furosemid, Nalidixinsäure, Haloperidol oder Captopril ist.

## 0 036 145

**Revendications pour les Etats Contractants: BE, CH, DE, FR, GB, IT, LI, LU, NL, SE**

1. Une formulation médicamenteuse pour l'emploi orale comprenant la combinaison d'un agent polaire bioactif avec un adjuvant comprenant un acide hydroxyarylique choisi parmi l'acide salicylique; l'acide 5 - méthoxysalicylique; l'acide 5 - méthylsalicylique; l'acide 5 - tert. - octylsalicylique; l'acide 3 - tert. - butyl - 6 - méthylsalicylique; l'acide 3 - tert. - butyl - 5 - méthylsalicylique; l'acide 5 - bromo - salicylique; l'acide 3,5-diiodosalicylique; l'acide 5 - trifluorométhyl - 2 - hydroxybenzoïque; l'acide 5 - butoxysalicylique et l'acide 5 - chlorosalicylique; ou un de leurs sels; en une quantité efficace pour accroître l'absorption orale de l'agent polaire bioactif, à l'exception de:
l'acide salicylique en combinaison avec:
(a) l'aminophénazone, la caféine et le 2 - méthyl - 6 - méthylheptène;
(b) la théophylline, l'acide phényléthylbarbiturique et la réserpine;
(c) l'acide acétylsalicylique;
(d) l'antipyrine, la caféine, l'opium et la coca;
(e) la théine et l'antipyrine;
(f) l'iodure de sodium plus la colchicine ou la scille ou l'aconit;
(g) l'antipyrine, le salicylamide, la caféine, la vitamine $B_1$ et l'adénosine;
(h) l'acétaminophène et la caféine;
(i) la phénylpropanolamine et la caféine;
(j) le bitartrate de dihydrocodéionone, le maléate de chlorotriméton, le citrate de sodium, la caféine et le gaïacolate de glycéryle;
(k) l'indométhacine;
(l) l'acide niflumique;
(m) la phénylbutazone;
(n) la 17-acétoxy - 6 - α - méthyl - 4 - α - prégnène - 3,20 - dione et/ou l'acétate de 19 - éthynyl - α - testostérone - énolcyclopentyle;
et à l'exception d'une combinaison de l'acide phénylquinoléine-carboxylique, l'acide acétylsalicylique, le salicylate de calcium, la caféine et la phénacétine.

2. La formulation médicamenteuse de la revendication 1 dans laquelle ledit agent polaire bioactif est choisi parmiles xanthines, les agents antitumoraux, les analgésiques narcotiques, un anion organique à activité biologique, les composés polyhydroxyliques bioactifs ou les polypeptides bioactifs.

3. La formulation médicamenteuse de la revendication 2, dans laquelle lesdites lesdites xanthines sont le triamtérène ou la théophylline.

4. La formulation médicamentuse de la revendication 2, dans laquelle ledit agent antitumoral est le 5 - fluoro - uridindésoxyriboside, le 6 - mercapto - purinedésoxyriboside ou la vidarabine.

5. La formulation médicamentuse de la revendication 2, dans laquelle ledit analgésique narcotique est l'hydromorphone, la cyclazine, la pentazocine ou la bupomorphine.

6. La formulation médicamenteuse de la revendication 2 dans laquelle ledit anion organique à activité biologique est l'héparine, la 15-méthylprostaglandine $E_2$, le cromolyn sodique ou la carbénoxolone.

7. La formulation médicamenteuse de la revendication 2 dans laquelle ledit composé polyhydroxylique bioactif est la dopamine, la dobutamine, la l-dopa ou l'α-méthyldopa.

8. La formulation médicamenteuse de la revendication 2 dans laquelle ledit polypeptide bioactif est l'acétate de saralasin, la bradykinine, l'insuline, l'ACTH, une enképhaline, une endorphine, la somatostatine ou la sécrétine.

9. La formulation médicamenteuse de la revendication 2 dans laquelle ledit agent polaire bioactif est la chlorotértracyline, la bromocriptine, la lidocaïne ou la cimétidine.

10. La formulation médicamenteuse de la revendication 2, dans laquelle ledit agent polaire bioactif est: l'amiloride, l'hydrochlorothiazide, le moduretic, le carbidopa, la lévodopa, la cyclobenzaprine, le diflunisal, l'indométhacine, la méthyldopa, le sulindac, le timolol, le chlorhydrate de (−) - 1 - (cyclopropylméthyl) - 4 - [3 - (trifluorométhylthio) - 5H - dibenzo[a,d]cycloheptène - 5 - ylidène] - pipéridine, le maléate de N - [(S) - 1 - (éthoxycarbonyl) - 3 - phénylpropyl] - L - alanyl - L - proline, l'oxalate de (+) - 10,11 - dihydro - 5 - méthyl - 5H - dibenzo[a,d]cycloheptène - 5,10 - imine, l'acide 1 - éthyl - 6 - fluoro - 1,4 - dihydro - 4 - oxo - 7 - (1 - pipérazinyl) - 3 - quinoléinecarboxylique, la 3 - fluoro - D - alanine et le sel de sodium hémihydrate de la D - 4 - (1 - méthyl - 3 - oxo - 1 - buténylamino) - 3 - isoxazolidinone, le L - N - (2 - oxopipéridine - 6 - ylcarbonyl - histidyl - L - thiazolidine - 4 - carboxamide, l'acide (6,7 - dichloro - 2 - méthyl - 2 - phényl - 1 - oxo - 5 - indanyloxy)acétique, l'ibuprofène, le naproxen, l'acide 5 - (4 - chlorobenzoyl) - 1,4 - diméthyl - 1H - pyrrole - 2 - acétique, la phénylbutazone, la dexaméthasone, la prednisolone, la clonidine, le propanolol, le diazépam, le chlorodiazépoxide, le furosémide, l'acide nalidixique, l'halopéridol et le captopril.

**Revendications pour l'Etat Contractant: AT**

1. Un procédé pour la préparation d'une formulation médicamenteuse pour l'emploi oral comprenant la combinaison d'un agent polaire bioactif avec un adjuvant comprenant un acid hydroxyarylique choisi parmi l'acide salicylique; l'acide 5 - méthoxysalicylique; l'acide 5 - méthylsalicylique; l'acide 5 - tert. -

octylsalicylique; l'acide 3 - tert. - butyl - 6 - méthylsalicylique; l'acide 3 - tert. - butyl - 5 - méthylsalicylique; l'acide 5 - bromosalicylique; l'acide 3,5 - diiodosalicylique; l'acide 5 - trifluorométhyl - 2 - hydroxybenzoïque; l'acide 5 - butoxysalicylique et l'acide 5 - chlorosalicylique; ou un de leurs sels; en une quantité efficace pour accroître l'absorption orale de l'agent polaire bioactif, à l'exception de: l'acide salicylique en combinaison avec:

(a) l'aminophénazone, la caféine et le 2 - méthyl - 6 - méthylheptène;

(b) la théophylline, l'acide phényléthylbarbiturique et la réserpine;

(c) l'acide acétylsalicylique;

(d) l'antipyrine, la caféine, l'opium et la coca,

(e) la théine et l'antipyrine;

(f) l'iodure de sodium plus la colchicine ou la scille ou l'aconit;

(g) l'antipyrine, le salicylamide, la caféine, la vitamine $B_1$ et l'adénosine;

(h) l'acétaminophène et la caféine;

(i) la phénylpropanolamine et la caféine;

(j) le bitartrate de dihydrocodéinone, le maléate de chlorotriméton, le citrate de sodium, la caféine et le gaïacolate de glycéryle;

(k) l'indométhacine;

(l) l'acide niflumique;

(m) la phénylbutazone;

(n) la 17 - acétoxy - 6 - α - méthyl - 4 - α - prégnène - 3,20 - dione et/ou l'acétate de 19 - éthynyl - α - testostérone - énolcyclopentyle;

et à l'exception d'une combinaison de l'acide phénylquinoléine-carboxylique, l'acide acétylsalicylique, le salicylate de calcium, la caféine et la phénacétine.

2. Le procédé de la revendication 1, dans lequel ledit agent polaire bioactif est choisi parmi les xanthines, les agents antitumoraux, les analgésiques narcotiques, un anion organique à activité biologique, les composés polyhydroxyliques bioactifs ou les polypeptides bioactifs.

3. Le procédé de la revendication 2, dans lequel lesdites xanthines sont le triamtérène ou la théophylline.

4. Le procédé de la revendication 2, dans lequel ledit agent antitumoral est le 5 - fluoro - uridinedésoxyriboside, le 6 - mercapto - purinedésoxyriboside ou la vidarabine.

5. Le procédé de la revendication 2, dans lequel ledit analgésique narcotique est l'hydromorphone, la cyclazine, la pentazocine ou la bupomorphine.

6. Le procédé de la revendication 2, dans lequel ledit anion organique à activité biologique est l'héparine, la 15 - méthylprostaglandine $E_2$, le cromolyn sodique ou la carbénoxolone.

7. Le procédé de la revendication 2, dans lequel ledit composé polyhydroxylique bioactif est la dopamine, la dobutamine, la l-dopa ou l'α-méthyldopa.

8. Le procédé de la revendication 2, dans lequel ledit polypeptide bioactif est l'acétate de saralasin, la bradykinine, l'insuline, l'ACTH, une enképhaline, une endorphine, la somatostatine ou la sécrétine.

9. Le procédé de la revendication 2, dans lequel ledit agent polaire bioactif est la chlorotétracycline, la bromocriptine, la lidocaïne ou la cimétidine.

10. Le procédé de la revendication 2, dans lequel ledit agent polaire bioactif est: l'amiloride, l'hydrochlorothiazide, le moduretic, le carbidopa, la lévodopa, la cyclobenzaprine, le diflunisal, l'indo-méthacine, la méthyldopa, le sulindac, le timolol, le chlorhydrate de (−) - 1 - (cyclopropylméthyl) - 4 - [3 - (trifluorométhylthio) - 5H - dibenzo[a,d]cycloheptène - 5 - ylidène] - pipéridine, le maléate de N - [(S) - 1 - (éthoxycarbonyl) - 3 - phénylpropyl] - L - alanyl - L - proline, l'oxalate de (+) - 10,11 - dihydro - 5 - méthyl - 5H - dibenzo[a,d]cycloheptène - 5,10 - imine, l'acide 1 - éthyl - 6 - fluoro - 1,4 - dihydro - 4 - oxo - 7 - (1 - pipérazinyl) - 3 - quinoléinecarboxylique, la 3 - fluoro - D - alanine et le sel de sodium hémihydrate de la D - 4 - (1 - méthyl - 3 - oxo - 1 - buténylamino) - 3 - isoxazolidinone, le L - N - (2 - oxopipéridine - 6 - ylcarbonyl) - histidyl - L - thiazolidine - 4 - carboxamide, l'acide (6,7 - dichloro - 2 - méthyl - 2 - phényl - 1 - oxo - 5 - indanyloxy) acétique, l'ibuprofène, le naproxen, l'acide 5 - (4 - chlorobenzoyl) - 1,4 - diméthyl - 1H - pyrrole - 2 - acétique, la phénylbutazone, la dexaméthasone, la prednisolone, la clonidine, le propanolol, le diazépam, le chlorodiazépoxide, le furosémide, l'acide nalidixique, l'halopéridol et le captopril.